# EUROPÄISCHE PATENTSCHRIFT

(11) **EP 1 955 994 B1**
(45) Veröffentlichungstag und Bekanntmachung des Hinweises auf die Patenterteilung: **05.05.2010**
(21) Anmeldenummer: 07002734.7
(22) Anmeldetag: 08.02.2007
(51) Int. Cl.: C07C 67/08, C07C 67/58, C07C 69/44

(54) **Verfahren zur Herstellung von DMA**
Method for manufacturing DMA
Procédé destiné à la fabrication de DMA

(43) Veröffentlichungstag der Anmeldung: 13.08.2008
(73) Patentinhaber: EMS-CHEMIE AG, 7013 Domat-Ems (CH)
(72) Erfinder: Gisler René, CH-7000 Chur (CH); Kaplan Andreas. Dr. rer. nat., CH-7000 Chur (CH)
(74) Vertreter: Pfenning, Meinig & Partner GbR

(56) Entgegenhaltungen:
- DE-A1- 2 330 235
- DE-A1- 4 241 448
- US-A- 4 076 948

## Beschreibung

Vorliegende Erfindung betrifft ein Verfahren zur Herstellung von DMA aus den Edukten Adipinsäure und Methanol.

Herstellungsverfahren zur Synthese von DMA sind bereits vielfach aus dem Stand der Technik bekannt. Den Verfahren betreffend die direkte Synthese aus Adipinsäure und Methanol ist gemein, dass unmittelbar nach dem Abschluss der Veresterungsreaktion eine Abtrennung des bei der Reaktion entstandenen Wassers erfolgt. Nachteilig dabei ist jedoch, dass somit zum Einen eine eigene Stufe zur Abtrennung des Wassers aus dem Reaktionsgemisch aufgewendet werden muss und zum Anderen, dass bei dieser Abtrennung von Wasser, die destillativ erfolgt, Nebenreaktionen auftreten können, die die Ausbeute verringern und die Aufreinigung des Produktes erschweren.

DE 2 330 235 offenbart ein Verfahren zur Gewinnung von Carbonsäureestern aus Abfallsalzlösungen der Cyclohexanonherstellung. Die Abfallsalzlösungen enthalten neben kleineren Mengen anderer Carbonsäuren, u.a. Adipinsäure, Valeriansäure und ε-Hydroxycarbonsäure. Nach dem Ansäuern der Abfalllösung wird mit einem niederigen Alkohol, vorzugsweise Methanol, verestert, gegebenenfalls in Gegenwart eines Lösungsmittels. Aus dem erhaltenen Estergemisch wird, gegebenenfalls nach Destillation des Lösungsmittels, eine Fraktion abgetrennt, die die Ester der Adipin- und ε-Hydroxycarbonsäure enthält, welche durch Extraktion mit Wasser in seine Komponenten zerlegt wird.

US 4,076,948 beschreibt die Wiedergewinnung von organischen Säuren, Salpetersäure bzw. Katalysatoren aus wässerigen, salpetersauren Lösungen, welche sich bei der Oxidation von Cyclohexanol und/oder Cyclohexanon ergeben. Dazu wird mit einem wasserlöslichen, niedrigen Alkylalkohol, z.B. Methanol, in Gegenwart eines nicht wassermischbaren Lösungsmittels verestert, die Produktester in die organische Phase extrahiert, die Phasen getrennt und das Lösungsmittel abdestilliert.

DE 42 41 448 A1 schildert ein Verfahren zur Herstellung schwefelfreier Dicarbonsäuredimethylester. Die Dicarbonsäure wird dabei mit überschüssigem Methanol in Gegenwart einer Alkylsulfonsäure als Katalysator bei 120 bis 180 °C verestert, Methanol und Wasser werden abdestilliert und das Sumpfprodukt wird ohne Aufarbeitung direkt einer fraktionierten Destillation unter Isolierung des Esters unterworfen.

Ausgehend hiervon ist es Aufgabe der vorliegenden Erfindung, ein Verfahren zur Herstellung von DMA aus Adipinsäure und Methanol anzugeben, das sich im Vergleich zu den im Stand der Technik genannten Verfahren durch eine vereinfachte Verfahrensführung und durch weitgehende Vermeidung von Nebenreaktionen und unerwünschten Nebenprodukten auszeichnet.

Diese Aufgabe wird durch das Verfahren mit den Merkmalen des Anspruchs 1 gelöst. Die abhängigen Ansprüche stellen dabei vorteilhafte Weiterbildungen dar.

Erfindungsgemäß zeichnet sich das Verfahren zur Herstellung von Dimethyladipat (DMA) durch Veresterung von Adipinsäure (ADS) mit Methanol (MeOH) durch die folgenden Schritte aus:
a) Umsetzung eines molaren Überschusses von MeOH mit ADS bei einer Temperatur größer Raumtemperatur bis zur Siedetemperatur des MeOH,
b) Neutralisation des Umsetzungsproduktes a) mit einem Alkalihydroxid,
c) Entfernen des MeOH aus dem MeOH/Wassergemisch durch Destillation und anschließende Zugabe von Xylol als Hilfslösungsmittel,
d) Abtrennung der wässrigen Phase enthaltend Monomethyladipat (MMA) vom Xylol,
e) Abtrennung des Xylols vom DMA durch Destillation und
f) Aufreinigung des DMA

Völlig überraschend hat sich gezeigt, dass sich Nebenprodukte oder -reaktionen fast vollständig vermeiden lassen, wenn das Reaktionswasser erst später im

Verfahren - also nicht unmittelbar nach der Veresterungsreaktion - abgetrennt wird. Ein wesentlicher Vorteil ist dabei, dass auf eine zusätzliche Destillationsstufe verzichtet werden kann. Vorteilhaft ist es dabei, wenn im Verfahrensschritt a) 1 bis 5 Mol ADS mit 5 bis 40 Mol MeOH, bevorzugt 1,5 bis 2,5 Mol ADS mit 15 bis 25 Mol MeOH umgesetzt werden.

Bevorzugt wird bei der Veresterungsreaktion in Schritt a) ein Katalysator zugesetzt, der die Reaktionsgeschwindigkeit sowie die Ausbeute erhöht. Dabei kann jeder im Stand der Technik bekannte Katalysator eingesetzt werden. Insbesondere wird bei der Umsetzung (Verfahrensschritt a)) 0,01 bis 0,8 Mol eines Katalysators, bevorzugt p-Toluolsulfonsäure-Monohydrat (p-TSA), zugesetzt.

Weiterhin ist es bevorzugt, wenn bei der Umsetzung in Schritt a) zusätzlich Xylol als Hilfslösungsmittel zugesetzt wird.

Die in Schritt a) beschriebene Umsetzungsreaktion wird dabei solange durchgeführt, bis ein Carboxylgruppenumsatz erreicht wird, der einen ökonomisch vorteilhaften Abbruch der Reaktion erlaubt. Vorteilhaft ist hierbei, wenn der Carboxylgruppenumsatz ≥ 90 %, bevorzugt ≥ 94 %, beträgt.

Die auf Schritt a) folgende Neutralisation, die z.B. mit beliebigen Laugen - insbesondere verdünnten, wässrigen Lösungen von Natriumhydroxid - ausgeführt werden kann, wird bevorzugt so durchgeführt, bis ein pH-Wert von 7,5 bis 8,5 erreicht ist.

Die im Verfahrensschritt c) ausgeführte destillative Abtrennung von MeOH erfolgt vorteilhafterweise unter vermindertem Druck, der insbesondere 100 bis 950 mbar, bevorzugt 250 mbar bis 600 mbar, besonders bevorzugt 400 bis 500 mbar, beträgt, unter Verwendung einer Kolonne mit mindestens zwei theoretischen Böden.

Weiterhin ist es bevorzugt, wenn die Abtrennung der wässrigen Phase vom Xylol (Verfahrensschritt d)) durch Absetzen erfolgt. Nach vollständig erfolgter Phasentrennung kann somit durch einfachste physikalische Trennmethoden (z.B. Dekantieren, Abpumpen etc.) eine Separation der wässrigen Phase erfolgen, was eine deutliche Vereinfachung der Verfahrensführung mit sich bringt. Die wässrige Phase enthält dabei das bei der Reaktion in geringen Anteilen anfaltende, aus unvollständigem Umsatz herrührende MMA.

Die Abtrennung des Xylols vom Produkt erfolgt ebenfalls bevorzugt durch Destillation, (Verfahrensschritt e)) bei einem Druck von kleiner als 400 mbar, bevorzugt kleiner als 200 mbar.

Die Aufreinigung des Rohprodukts im Verfahrensschritt f) erfolgt bevorzugt durch Filtration.

Eine besonders ökonomische und ökologische Verfahrensführung ergibt sich, wenn das durch Destillation abgetrennte Methanol in Verfahrensschritt c) in den Verfahrensschritt a) zurückgeführt wird. Das gesagte gilt auf für das im Verfahrensschritt e) zurückgenommene Xylol, das ebenfalls nach der Destillation entweder zu Verfahrensschritt a) und/oder c) zugegeben werden kann.

Unter den gleichen Gesichtspunkten ist es ebenso vorteilhaft, wenn aus der beim Verfahrensschritt d) abgetrennten wässrigen Phase das Monomethyladipat (MMA) aufgearbeitet und in den Verfahrensschritt a) zur Weiterveresterung zurückgeführt wird.

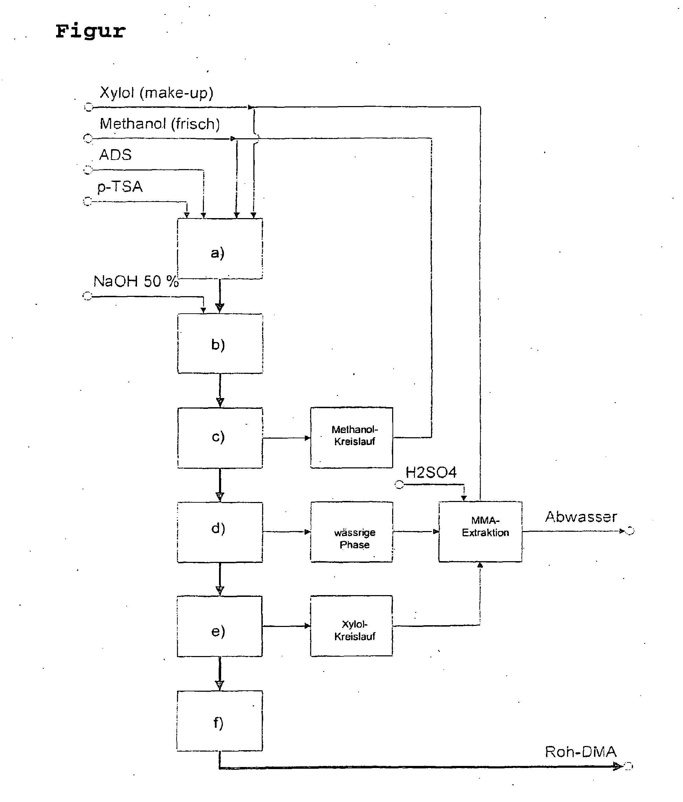

Die vorliegende Erfindung wird anhand des nachfolgenden Ausführungsbeispiels näher erläutert, ohne die Erfindung auf die dort genannten spezifischen Verfahrensparameter einzuschränken. Dazu wird auch auf das in Fig. 1 abgebildete Fliesschema Bezug genommen.

Die Versuchsapparatur besteht im Wesentlichen aus einem 2'000 mL 4-Halsrundkolben mit Bodenventil und Heizpilz. Der Reaktionskolben ist mit einem Metallrührer, einer ca. 35 cm langen Füllkörperkolonne mit Maschendrahtwendel und einem Kolonnenkopf bestückt. Als Kondensator wird ein 20 cm langer Intensivkühler und als Destillatkühler ein Spiralkühler verwendet. Nach dem Destillatkühler befindet sich ein Anschütz-Thiele-Vorstoss, um während den beiden Vakuumdestillationen Destillatproben zu entnehmen. Die Ansatzgröße erfolgt dabei mit den in Tabelle 1 aufgeführten Stoffen und Mengen.

**Tabelle 1**

| Stoff | Stoffmenge | |
|---|---|---|
| | [g] | [mol] |
| Adipinsäure (ADS) | 292.00 | 2.000 |
| Methanol (MeOH) | 640.00 | 20.000 |
| p-Toluolsulfonsäure Monohydrat (p-TSA) | 8.24 | 0.043 |
| Xylol [mL] | 200 | - - - |
| NaOH 50 % ¹⁾ | 20.05 | - - - |
| H₂SO₄ 20 % ²⁾ | 55.08 | - - - |

| | | |
|---|---|---|
| ¹⁾ Notwendige Menge für die Einstellung eines pH-Wertes von 8.0 (Mittelwert aus 8 Laborversuchen) ²⁾ Notwendige Menge für die Einstellung eines pH-Wertes von 2.0 (Mittelwert aus 3 Laborversuchen). Um die wässrige Phase der MMA-Extraktion nicht zu stark zu verdünnen, müsste eine höher konzentrierte Schwefelsäure-Lösung eingesetzt werden. | | |

### 1. Veresterung (Umgebungsdruck) (Verfahrensschritt a))

P-TSA wird in ca. 10 mL MeOH gelöst. ADS und das restliche MeOH werden im Reaktionskolben vorgelegt und auf 55 °C aufgeheizt. Sobald die ADS vollständig gelöst ist (ca. 30 Minuten), wird die p-TSA-Lösung zugegeben. Dann wird die Temperatur auf 70 °C erhöht (Siedetemperatur). Bei dieser Temperatur wird während 2 Stunden verestert. Nach dieser Zeit ist der Carboxylgruppen-Umsatz konstant und liegt bei ≥ 94 %. Der Carboxylgruppen-Umsatz wurde aus der titrimetrischen Bestimmung (0.1 n NaOH) der Carboxylgruppen-Konzentration eines dem Reaktionsgemisch entnommenen Aliquotes berechnet. Das Reaktionsgemisch wird auf 50 °C abgekühlt. Das Verhältnis DMA/MMA liegt bei etwa 91/9.

### 2. Neutralisation (Umgebungsdruck) (Verfahrensschritt b))

Zur pH-Messung wird eine kombinierte Glaselektrode in den Reaktionskolben eingebaut. Bei einer Temperatur von 50 °C wird durch Zugabe der 50 %igen NaOH-Lösung ein pH-Wert von 8.0 ± 0.1 eingestellt. Die Zugabe erfolgt innerhalb von ca. 10 Minuten. Anschließend wird die pH-Elektrode wieder ausgebaut. Die Starttemperatur bei der MeOH-Destillation liegt bei etwa 51 bis 52 °C, deshalb sollte die Neutralisationstemperatur nicht über 50 °C sein.

### 3. MeOH-Destillation (450 mbar) (Verfahrensschritt c))

Nach der Neutralisation wird der Reaktordruck auf 450 mbar abgesenkt und das Gemisch zum Sieden erhitzt. Die Reaktortemperatur liegt anfänglich bei 51 bis 52 °C und wird dann langsam auf etwa 80 °C gesteigert. Während dieser Zeit wird die Hauptmenge des MeOHs (durchschnittlich 481 g oder etwa 94 % MeOH-Kreislauf mit einem H₂O-Gehalt von 0.2 bis 0.3 %) bei einer konstanten Kopftemperatur von 45 bis 46 °C abdestilliert. Bei einer Reaktortemperatur von ≥ 80 °C steigt die Kopftemperatur in kurzer Zeit auf über 70 °C an. Bei Erreichen einer Reaktortemperatur von 83 °C wird die Destillation abgebrochen. Das dabei anfallende Destillat wird separat aufgefangen (durchschnittlich 17.6 g mit einem H₂O-Gehalt von etwa 10 bis 20 %). Der Destillationsrückstand wird auf 50 °C abgekühlt und dann das Vakuum mit Stickstoff gebrochen. Nun wird das Xylol zugegeben (siehe oben) und das Gemisch auf 50 °C temperiert.

### 4. Phasentrennung (Umgebungsdruck) (Verfahrensschritt d))

Nach der Xylol-Zugabe und Temperierung des Gemischs auf 50 °C wird die Rührung ausgeschaltet. Nach einer Phasentrennzeit von 5 bis 10 Minuten (scharfe Phasentrennschicht) werden durchschnittlich etwa 120 g wässrige Phase abgetrennt (enthält ca. 17 bis 22 % MMA in Form des Natriumsalzes und 5 bis 8 % DMA;
Rest; Wasser und anorganische Salze).

### 5. MMA-Extraktion/Phasentrennung (Umgebungsdruck)

Die wässrige Phase (ca. 120 g) wird zusammen mit 100 mL Xylol im Reaktionskolben vorgelegt und auf 50 °C erwärmt. Zur pH-Messung wird eine kombinierte Glaselektrode in den Reaktionskolben eingebaut. Durch Zugabe einer 20 %igen H₂SO₄-Lösung wird der pH-Wert auf 2.0 ± 0.1 eingestellt. Dann wird während 5 Minuten intensiv gemischt. Nach einer Phasentrennzeit von 5 bis 10 Minuten wird die untere wässrige Phase abgetrennt. Die organische Phase wird aus dem Reaktionskolben entfernt. Die abgetrennte wässrige Phase wird ein zweites Mal mit 100 mL Xylol extrahiert. Die vereinigten organischen Phasen können nun in den Prozess zurückgeführt werden. Bei den Laborversuchen wurden sie zusätzlich über Na₂SO₄ getrocknet, filtriert und dann zur Trockene eingedampft, was jedoch keinerlei Auswirkungen auf den Verfahrensverlauf sowie die Ausbeute des Verfahrens hat; ebenso kann diese Trocknungsstufe ausgelassen werden.

Dabei wurden zwischen 30 und 35 g Eindampfrückstand erhalten, der sich aus 70 bis 75 % MMA und 20 bis 27 % DMA zusammensetzt.

Wie bereits unter Pkt. 2 erwähnt, sollte für die Neutralisation eine höher konzentrierte Schwefelsäure-Lösung eingesetzt werden, um die wässrige Phase weniger stark zu verdünnen und so die Ausbeute bei der Extraktion zu erhöhen.

### 6. Xylol-Destillation (150 → 50 mbar) (Verfahrensschritt e))

Nach der Abtrennung der wässrigen Phase (vgl. 3.) wird der Reaktordruck auf 150 mbar reduziert und das Gemisch (Roh-DMA, Xylol und Rest-Wasser) zum Sieden erhitzt. Bei einer Kopftemperatur von 45 bis 50 °C wird die Hauptmenge des Xylols zusammen mit dem im Reaktionsgemisch gelösten Wasser abdestilliert. Bei einer Reaktortemperatur von 90 °C wird der Druck von 150 auf 50 mbar abgesenkt und die Destillation bis zu einer Reaktortemperatur von 130 °C weiter betrieben. Dann wird auf Raumtemperatur abgekühlt und das Vakuum mit Stickstoff gebrochen. Es werden durchschnittlich 299 g Roh-DMA mit ≥ 99 % DMA, ≤ 1 % MMA und einem H₂O-Gehalt von ≤ 0.05 % erhalten. Somit ist das erhaltene Roh-DMA bereits von so hoher Qualität bzw. enthält so wenig Nebenprodukte, dass weitere aufwändige Aufreinigungsstufen, wie beispielsweise destillative Aufreinigungen, entfallen können. Die Gesamtausbeute setzt sich zusammen aus den 299 g Roh-DMA (ca. 85 % der Theorie) und den ca. 30 bis 35 g Eindampfrückstand (vgl. 5.), die wieder in die Veresterung zurückgeführt werden können und aus denen etwa 30 bis 35 g Roh-DMA entstehen (ca. 8 bis 10 % der Theorie). Das bei der Destillation anfallende Xylol kann für die MMA-Extraktion eingesetzt werden.

### 7. Klärfiltration DMA (Verfahrensschritt f))

Im Roh-DMA scheiden sich beim Abkühlen noch wenig Rest-Salze aus, die im Rohprodukt eine schwache Trübung verursachen. Die Mengen sind gering (< 0.2 %). Die Filtrierbarkeit ist gut. Bei den Laborversuchen erfolgte die Klärfiltration mit einem Filterhilfsmittel (FibraFlow).

Die abschließend erhaltenen Produkteigenschaften sind zusammenfassend in Tabelle 2 aufgeführt.

**Tabelle 2**

| Kennzahl | Wert |
|---|---|
| Reinheit (GC) [F1-%] | ≥ 99.0 |
| Wassergehalt [%] | ≤ 0.05 |
| Monomethyladipat [%] | ≤ 1.0 |
| Farbzahl [APHA] | ≤ 20 |
| Säurezahl [mg KOH/g] | ≤ 3.5 |

## Patentansprüche

1. Verfahren zur Herstellung von Dimethyladipat (DMA) durch Veresterung von Adipinsäure (ADS) mit Methanol (MeOH) mit folgenden Schritten:
a) Umsetzung eines molaren Überschusses von MeOH mit ADS bei einer Temperatur größer Raumtemperatur bis zur Siedetemperatur des MeOH,
b) Neutralisation des Umsetzungsproduktes a) mit einem Alkalihydroxid,
c) Entfernen des MeOH aus dem MeOH/wassergemisch durch Destillation und anschließende Zugabe von Xylol als Hilfslösungsmittel,
d) Abtrennung der wässrigen Phase enthaltend Monomethyladipat (MMA) vom Xylol,
e) Abtrennung des Xylols vom DMA durch Destillation und
f) Aufreinigung des DMA.

2. Verfahren nach Anspruch 1,
**dadurch gekennzeichnet, dass** beim Verfahrensschritt a) 1 bis 5 Mol ADS mit 5 bis 40 Mol MeOH umgesetzt werden.

3. Verfahren nach Anspruch 1 oder 2,
**dadurch gekennzeichnet, dass** bei der Umsetzung (Verfahrensschritt a)) 0,01 bis 0,8 Mol eines Katalysators, bevorzugt p-ToluolsulfonsäureMonohydrat (p-TSA), zugesetzt wird.

4. Verfahren nach einem der Ansprüche 1 bis 3,
**dadurch gekennzeichnet, dass** bei der Umsetzung in Schritt a) zusätzlich Xylol als Lösungsmittel zugesetzt wird.

5. Verfahren nach einem der Ansprüche 1 bis 4,
**dadurch gekennzeichnet, dass** die Umsetzung so lange durchgeführt wird, bis ein Carboxylgruppenumsatz ≥ 90 %, bevorzugt ≥ 94 %, erreicht ist.

6. Verfahren nach mindestens einem der Ansprüche 1 bis 5,
**dadurch gekennzeichnet, dass** die Neutralisation (Verfahrensschritt b)) durchgeführt wird, bis ein pH-Wert von 7,5 bis 8,5 erreicht ist.

7. Verfahren nach Anspruch 6,
**dadurch gekennzeichnet, dass** die Neutralisation mit einer verdünnten NaOH-Lösung durchgeführt wird.

8. Verfahren nach mindestens einem der Ansprüche 1 bis 7,
**dadurch gekennzeichnet, dass** die Destillation (Verfahrensschritt c)) bei einem Druck von 100 mbar bis 950 mbar mit einer Kolonne mit mindestens zwei Böden durchgeführt wird.

9. Verfahren nach mindestens einem der Ansprüche 1 bis 8,
**dadurch gekennzeichnet, dass** die Abtrennung der wässrigen Phase vom Xylol (Verfahrensschritt d)) durch Absetzen erfolgt.

10. Verfahren nach mindestens einem der Ansprüche 1 bis 9,
**dadurch gekennzeichnet, dass** die Abtrennung des Xylols durch Destillation (Verfahrensschritt e)) bei einem Druck von kleiner als 400 mbar erfolgt.

11. Verfahren nach mindestens einem der Ansprüche 1 bis 10,
**dadurch gekennzeichnet, dass** die Aufreinigung des DMA (Verfahrensschritt f)) durch Filtration erfolgt.

12. Verfahren nach mindestens einem der Ansprüche 1 bis 10,
**dadurch gekennzeichnet, dass** das durch Destillation abgetrennte Methanol in Verfahrensschritt c)) in den Verfahrensschritt a)) zurückgeführt wird.

13. Verfahren nach mindestens einem der Ansprüche 1 bis 12,
**dadurch gekennzeichnet, dass** aus der beim Verfahrensschritt d) abgetrennten wässrigen Phase das Monomethyladipat (MMA) abgetrennt und in den Verfahrensschritt a) zurückgeführt wird.

14. Verfahren nach mindestens einem der Ansprüche 1 bis 13,
**dadurch gekennzeichnet, dass** das beim Verfahrensschritt e) abgetrennte Xylol in die Verfahrensschritten a) und/oder c) zurückgeführt wird.

## Claims

1. Process for the preparation of dimethyl adipate (DMA) by esterification of adipic acid (ADA) with methanol (MeOH) having the following steps:
a) reaction of a molar excess of MeOH with ADA at a temperature greater than room temperature up to the boiling temperature of the MeOH,
b) neutralisation of the reaction product a) with an alkali hydroxide,
c) removal of the MeOH from the MeOH/water mixture by distillation and subsequent addition of xylene as auxiliary solvent,
d) separation of the aqueous phase containing monomethyl adipate (MMA) from the xylene,
e) separation of the xylene from the DMA by distillation, and
f) purification of the DMA.

2. Process according to Claim 1,
**characterised in that** in process step a) 1 to 5 mol of ADA are reacted with 5 to 40 mol of MeOH.

3. Process according to Claim 1 or 2,
**characterised in that** in the reaction (process step a)) 0.01 to 0.8 mol of a catalyst, preferably p-toluenesulphonic acid monohydrate (p-TSA), is added.

4. Process according to one of Claims 1 to 3,
**characterised in that** in the reaction in step a) xylene is additionally added as solvent.

5. Process according to one of Claims 1 to 4,
**characterised in that** the reaction is carried out until a carboxyl group conversion of ≥ 90%, preferably ≥ 94%, is reached.

6. Process according to at least one of Claims 1 to 5,
**characterised in that** the neutralisation (process step b)) is carried out until a pH value of 7.5 to 8.5 is reached.

7. Process according to Claim 6,
**characterised in that** the neutralisation is carried out using a dilute NaOH solution.

8. Process according to at least one of Claims 1 to 7,
**characterised in that** the distillation (process step c)) is carried out at a pressure of 100 mbar to 950 mbar using a column with at least two plates.

9. Process according to at least one of Claims 1 to 8,
**characterised in that** the separation of the aqueous phase from the xylene (process step d)) is performed by sedimentation.

10. Process according to at least one of Claims 1 to 9,
**characterised in that** the separation of the xylene by distillation (process step e)) is performed at a pressure of less than 400 mbar.

11. Process according to at least one of Claims 1 to 10,
**characterised in that** the purification of the DMA (process step f)) is performed by filtration.

12. Process according to at least one of Claims 1 to 10,
**characterised in that** the methanol separated by distillation in process step c)) is fed back to process step a)).

13. Process according to at least one of Claims 1 to 12,
**characterised in that** the monomethyl adipate (MMA) is separated from the aqueous phase separated in process step d) and is fed back to process step a).

14. Process according to at least one of Claims 1 to 13,
**characterised in that** the xylene separated in process step e) is fed back to process steps a) and/or c).

## Revendications

1. Procédé de fabrication d'adipate de diméthyle (DMA) par estérification d'acide adipique (ADS) avec du méthanol (MeOH), comprenant les étapes consistant à :
a) faire réagir un excès molaire de MeOH avec de l'ADS à une température supérieure à la température ambiante, jusqu'à la température d'ébullition du MeOH,
b) neutraliser le produit de la réaction obtenu à l'étape a) avec un hydroxyde de métal alcalin,
c) éliminer le MeOH du mélange de MeOH/eau par distillation et ajouter ensuite du xylène comme solvant auxiliaire,
d) séparer la phase aqueuse contenant l'adipate de monométhyle (MMA) du xylène,
e) séparer le xylène du DMA par distillation, et
f) purifier le DMA.

2. Procédé selon la revendication 1,
**caractérisé en ce que** l'on fait réagir, à l'étape de procédé a), 1 à 5 moles d'ADS avec 5 à 40 moles de MeOH.

3. Procédé selon la revendication 1 ou 2,
**caractérisé en ce que** l'on ajoute, lors de la réaction (étape de procédé a)), 0,01 à 0,8 mole d'un catalyseu
r, de préférence, du monohydrate d'acide p-toluènesulfonique (p-TSA).

4. Procédé selon l'une quelconque des revendications 1 à 3, **caractérisé en ce que** l'on ajoute, lors de la réaction réalisée à l'étape a), en outre du xylène comme solvant.

5. Procédé selon l'une quelconque des revendications 1 à 4, **caractérisé en ce que** l'on poursuit la réaction jusqu'à ce que l'on ait atteint une conversion des groupements carboxyle ≥ 90 %, de préférence ≥ 94 %.

6. Procédé selon au moins l'une quelconque des revendications 1 à 5,
**caractérisé en ce que** l'on réalise la neutralisation (étape de procédé b)) jusqu'à ce que l'on ait atteint une valeur de pH de 7,5 à 8,5.

7. Procédé selon la revendication 6,
**caractérisé en ce que** l'on réalise la neutralisation avec une solution de NaOH diluée.

8. Procédé selon au moins l'une quelconque des revendications 1 à 7,
**caractérisé en ce que** l'on réalise la distillation (étape de procédé c)) à une pression de 100 mbars à 950 mbars avec une colonne comprenant au moins deux plateaux.

9. Procédé selon au moins l'une quelconque des revendications 1 à 8,
**caractérisé en ce que** la séparation de la phase aqueuse du xylène (étape de procédé d)) se fait par sédimentation.

10. Procédé selon au moins l'une quelconque des revendications 1 à 9,
**caractérisé en ce que** la séparation du xylène par distillation (étape de procédé e)) se fait à une pression inférieure à 400 mbars.

11. Procédé selon au moins l'une quelconque des revendications 1 à 10,
**caractérisé en ce que** la purification du DMA (étape de procédé f)) se fait par filtration.

12. Procédé selon au moins l'une quelconque des revendications 1 à 10,
**caractérisé en ce que** le méthanol séparé par distillation à l'étape de procédé c)) est renvoyé dans l'étape de procédé a)).

13. Procédé selon au moins l'une quelconque des revendications 1 à 12,
**caractérisé en ce que** l'adipate de monométhyle (MMA) est séparé de la phase aqueuse séparée lors de l'étape de procédé d) et est renvoyé dans l'étape de procédé a).

14. Procédé selon au moins l'une quelconque des revendications 1 à 13,
**caractérisé en ce que** le xylène séparé lors de l'étape de procédé e) est renvoyé dans les étapes de procédé a) et/ou c).
